# EUROPEAN PATENT APPLICATION

(11) **EP 3 360 592 A1**
(43) Date of publication of application: **15.08.2018**
(21) Application number: 17782569.2
(22) Date of filing: 23.02.2017
(51) Int. Cl.: A61M 5/32

(54) **SYRINGE-COUPLED SAFETY ASSEMBLY AND SAFETY SYRINGE INCLUDING SAME**

(30) Priority: 11.04.2016 KR 20160044303
(71) Applicant: Kim, Jung Gyu, Seoul 06090 (KR); Jang, Hong Sun, Seoul 06574 (KR)
(72) Inventor: Kim, Jung Gyu, Seoul 06090 (KR); Jang, Hong Sun, Seoul 06574 (KR)
(74) Representative: Grosse Schumacher Knauer von Hirschhausen
(86) International application number: PCT/KR2017/001963
(87) International publication number: WO 2017/179813

(57) **Abstract**

The present invention relates to a syringe-coupled safety assembly and a safety syringe including the same. According to one aspect of the present invention, provided is the syringe-coupled safety assembly comprising: a cover having a space part for allowing a syringe to enter therein, and a first through-hole through which a syringe needle passes; and a fixing unit having a second through-hole into which a syringe hub, in which the needle is mounted, is inserted, disposed inside the cover so as to be capable of proceeding in the direction becoming closer to the first through-hole and of retracting in the direction becoming farther from the first through-hole, fixed to the cover in a proceeded state, and provided so as to allow the syringe inserted into the second through-hole to retract when the fixed state thereof of the cover is released from the proceeded state.

## Description

### [Technical Field]

The present invention relates to a syringe-coupled safety assembly and a safety syringe including the same, and more particularly, to a syringe-coupled safety assembly capable of preventing piercing of the needle of a syringe and blocking contaminants, and a safety syringe including the same.

### [Background Art]

In general, a syringe is used for blood sampling or for suction or injection of a medical solution. Recently, attention has been increasingly drawn to safety syringes for protecting practitioners from possible infection caused by accidental puncture, which may occur by mistake during medical practices.

As a conventional method for safety treatment of a syringe needle, the syringe needle is inserted into and stored in a syringe cap. However, in the insertion process, practitioners often unintentionally pierce their hands with the syringe needle.

Therefore, there is a need for a safety syringe which can secure safety of the syringe needle, is easy to operate, and can reduce manufacturing costs. Further, when use of the syringe needle is finished, it is necessary to seal the syringe needle from the external environment in order to block contaminants.

### [Disclosure]

### [Technical Problem]

It is an object of the present invention to provide a syringe-coupled safety assembly capable of preventing injury and infection caused by a syringe needle, and a safety syringe including the same.

It is another object of the present invention to provide a syringe-coupled safety assembly which can be selectively connected to and disconnected from a syringe according to the use status of the syringe in order to improve usability, and a safety syringe including the same.

It is yet another object of the present invention to provide a syringe-coupled safety assembly capable of quickly and conveniently switching the needle of a syringe that has been used for a procedure to a stored state, and a safety syringe including the same.

It is yet another object of the present invention to provide a syringe-coupled safety assembly capable of sealing a needle in a stored state from the external environment after a procedure is completed, and a safety syringe including the same.

### [Technical Solution]

In accordance with one aspect of the present invention, provided is a syringe-coupled safety assembly comprising a cover having a space for entry of a syringe and a first through hole allowing a needle of the syringe to pass therethrough; and a fixing unit having a second through hole into which a hub of the syringe equipped with the needle is inserted, the fixing unit being disposed inside the cover so as to advance toward the first through hole and retreat from the first through hole and configured to be fixed to the cover in an advanced state and cause the syringe inserted into the second through hole to retreat when released from a fixed state with respect to the cover in the advanced state.

In accordance with another aspect of the present invention, provided is a safety syringe comprising a syringe comprising a housing, a hub mounted on the housing, a needle mounted on the hub, a plunger disposed in the housing, and a plunger rod connected to the plunger; and a syringe-coupled safety assembly detachably mounted on the syringe to protect the needle from an outside.

Herein, the syringe-coupled safety assembly comprises a cover having a space for entry of the syringe and a first through hole allowing the needle of the syringe to pass therethrough; and a fixing unit having a second through hole into which the hub of the syringe equipped with the needle is inserted, the fixing unit being disposed inside the cover so as to advance toward the first through hole and retreat from the first through hole and configured to be fixed to the cover in an advanced state and cause the syringe inserted into the second through hole to retreat when released from a fixed state with respect to the cover in the advanced state.

In accordance with yet another aspect of the present invention, provided is a syringe-coupled safety assembly comprising a cover having a space for entry of a syringe and a first through hole allowing a needle of the syringe to pass therethrough; a sealing member provided in the first through hole, the sealing member being configured to allow the needle of the syringe to pass therethrough; and a fixing unit having a second through hole into which a hub of the syringe equipped with the needle is inserted, the fixing unit being disposed inside the cover so as to advance toward the first through hole and retreat from the first through hole and configured to be fixed to the cover in an advanced state according to advance of the syringe in the space and cause the syringe to retreat when released from a fixed state with respect to the cover in the advanced state, wherein the sealing member is configured to move along the first through hole in an advancing direction when the needle passes therethrough during advance of the syringe and to move along the first through hole in a retreating direction when the needle passes therethrough during retreat of the syringe.

In accordance with yet another aspect of the present invention, provided is a safety syringe comprising a syringe comprising a housing, a hub mounted on the housing, a needle mounted on the hub, a plunger disposed in the housing, and a plunger rod connected to the plunger; and a syringe-coupled safety assembly detachably mounted on the syringe to selectively accommodate the needle.

Herein, the syringe-coupled safety assembly comprises: a cover having a space for entry of a syringe and a first through hole allowing a needle of the syringe to pass therethrough; a sealing member provided in the first through hole, the sealing member being configured to allow the needle of the syringe to pass therethrough; and a fixing unit having a second through hole into which a hub of the syringe equipped with the needle is inserted, the fixing unit being disposed inside the cover so as to advance toward the first through hole and retreat from the first through hole and configured to be fixed to the cover in an advanced state according to advance of the syringe in the space and cause the syringe to retreat when released from a fixed state with respect to the cover in the advanced state. In addition, the sealing member is configured to seal the first through hole when the needle passes during retreat of the syringe.

### [Advantageous Effects]

As is apparent from the foregoing, a syringe-coupled safety assembly and a safety syringe including the same related to an embodiment of the present invention have the following effects.

The needle of a syringe can be quickly and conveniently switched to a stored state after a procedure related to blood sampling, and suction and injection of a medical solution is completed. In addition, the syringe-coupled safety assembly may be selectively connected to and disconnected from the syringe according to the use status of the syringe in order to improve usability. Therefore, injury and infection caused by the syringe needle may be prevented.

Further, after the procedure is completed, the needle may be sealed from the external environment through the sealing member formed of silicone or rubber (e.g., urethane or the like) when the needle is stored.

### [Description of Drawings]

FIG. 1 is a cross-sectional view of a safety syringe related to an embodiment of the present invention.
FIGS. 2 to 4 are cross-sectional views illustrating an operational state of the syringe-coupled safety assembly shown in FIG. 1.
FIGS. 5 to 10 are cross-sectional views illustrating an operational state of the safety syringe shown in FIG. 1.
FIG. 11 is a plan view illustrating a guide rail and a fixing body.

### [Best Mode]

Hereinafter, a syringe-coupled safety assembly and a safety syringe including the same according to an embodiment of the present invention will be described in detail with reference to the accompanying drawings.

The same or similar reference numerals are assigned to the same or corresponding elements throughout the drawings, and redundant description thereof will be omitted. For simplicity, the size and shape of each constituent member shown in the drawings can be exaggerated or reduced.

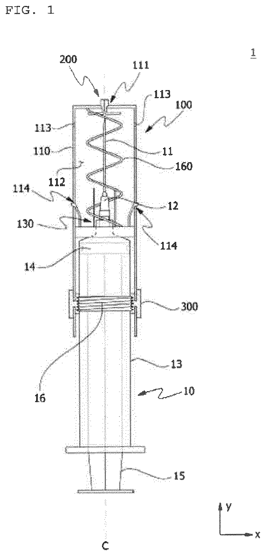

FIG. 1 is a cross-sectional view of a safety syringe 1 related to an embodiment of the present invention.

The safety syringe 1 related to an embodiment of the present invention includes a syringe 10 and a syringe-coupled safety assembly 100. The syringe 10 is a typical syringe that is widely used. For example, the syringe 10 includes a housing 13, a hub 12 mounted on the housing 13, a needle 11 mounted on the hub 12, a plunger 14 disposed in the housing 13, and a plunger rod 15 connected to the plunger 14.

In this embodiment, the syringe-coupled safety assembly 100 is detachably mounted on the syringe 10 to protect the needle 11 of the syringe 10 from the outside. Specifically, the syringe-coupled safety assembly 100 is detachably mounted on the syringe 10 to selectively accommodate the needle 11 of the syringe 10.

Referring to FIG. 1, the syringe-coupled safety assembly 100 includes a cover 110 and a fixing unit 130. The symbol C in FIG. 1, which is not described above, represents the central axis of the cover 111. The central axis of the safety syringe 1 may be the same as the central axis of the cover 110. In this specification, advance and retreat of each component may refer to movement along the y-axis of FIG. 1 parallel to the central axis C. In addition, the radial direction of each component may refer to the direction of the x-axis of FIG. 1.

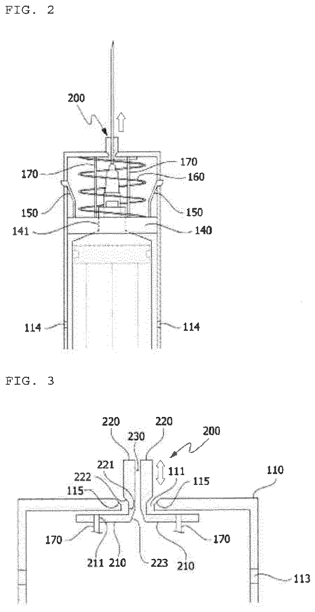

FIGS. 2 to 4 are cross-sectional views illustrating an operational state of the syringe-coupled safety assembly 100 shown in FIG. 1.

Referring to FIGS. 2 to 4, the syringe-coupled safety assembly 100 includes a cover 110 having a space 112 for entry of the syringe 10 and a first through hole 111 allowing the needle 11 of the syringe 10 to pass therethrough, and a fixing unit 130 having a second through hole 141 into which the hub 12 of the syringe 10 equipped with the needle 11 is inserted, the fixing unit 130 being disposed inside the cover 110 so as to advance toward the first through hole 111 and retreat from the first through hole 111 and configured to be fixed to the cover 110 in an advanced state and cause the syringe inserted into the second through hole 141 to retreat when released from a fixed state with respect to the cover 110 in the advanced state. Here, the first through hole 111 may be positioned on the central axis C of the cover 110. In addition, the first through hole 111 and the second through hole 141 may be coaxially arranged.

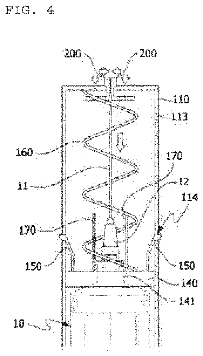

Referring to FIGS. 2 and 3, when the fixing unit 130 is in the advanced state, the needle 11 of the syringe 10 may be exposed to the outside through the first through hole 111. In addition, referring to FIG. 4, when the fixing unit 130 is in the retreated state, the needle of the syringe may be positioned inside the cover 110 through the first through hole 111. That is, the advanced state of the fixing unit 130 means that the needle 11 is exposed to the outside of the syringe-coupled safety assembly 100, and a procedure such as blood sampling or injection can be performed through the needle 11. In contrast, the retreated state of the fixing unit 130 means that the needle 11 has retreated into the cover 110 after the procedure is completed. In the retreating process, the fixing unit 130 retreats together with the syringe 10 inserted into the second through hole 141.

That is, the fixing unit 130 is arranged to advance and retreat within the cover 110 according to operation of the user. The fixing unit 130 may be detachably connected (coupled) to the syringe 10. In addition, when the syringe 10 is inserted into the fixing unit 130, and is thus coupled to the fixing unit 130, the fixing unit 130 and the syringe 10 are moved together.

Hereinafter, a fixing unit 130 for causing the needle 11 of the syringe 10 to retreat will be described in detail with reference to the accompanying drawings.

Referring to FIG. 2, the fixing unit 130 may include a first plunger 140, one or more fixing members 150, and an elastic member 160. Specifically, the first plunger 140 has a second through hole 141. In addition, the first plunger 140 is sealably and slidably disposed inside the cover 111. The fixing members 150 are connected to the first plunger 140, and are selectively fixed to the cover 111. In addition, one or more, namely a plurality of, fixing members 150 may be provided. For example, the plurality of fixing members 150 may be arranged spaced apart from each other by a predetermined distance in a circumferential direction of the cover 110. For example, the fixing members 150 may have a bar shape, and at least one region of the fixing member 150 may be bent. In addition, the elastic member 160 is disposed between the first plunger 140 and the first through hole 111.

The cover 110 may have a first fixing through hole 113 into which the fixing member 150 is inserted when the fixing unit 130 is in an advanced state. In addition, the cover 110 may have a second fixing hole 114 into which the fixing member is inserted when the fixing unit 130 is in a retreated state. The first fixing hole 113 and the second fixing hole 114 may be spaced apart from each other by a predetermined distance. The distance between the first fixing hole 113 and the second fixing hole 114 may be properly determined in consideration of the length of the needle 11 and the like. Alternatively, the cover 110 may have only the first fixing hole 113.

The elastic member 160 may be fixed to the cover 110 and the first plunger 140. For example, the elastic member 160 may be a coil spring. In addition, the elastic member 160 may be arranged to apply restoring force in a direction in which the fixing unit 130 retreats. That is, when the fixing unit 130 advances and is fixed to the first fixing hole 113 (hereinafter referred to as "advanced state"), the elastic member 160 is compressed in the direction of the y-axis. When the fixing unit 130 is separated from the first fixing hole 113 and retreats, the elastic member 160 is stretched along the y-axis, thereby being restored to the original state.

At least one region of the fixing member 150 may be bent to exert elastic force in a radial direction (x-axis direction) with respect to the central axis C of the cover 110. Specifically, when the fixing member 150 is inserted into and fixed to the first fixing hole 113, a region of the fixing member 150 is exposed to the outside of the first fixing hole 113.

At this time, if external force is exerted in the radial direction by the user, the fixing member 150 is separated from the first fixing hole 113, and the entire region of the fixing member 150 is positioned inside the cover 111. In addition, as the fixing member 150 is separated from the first fixing hole 113, the fixing unit 130 is caused to retreat by the restoring force of the elastic member 160. When the entire region of the fixing member 150 is positioned inside the cover 111, the fixing member 150 is compressed in the radial direction of the cover 110.

Thereafter, when the compressive force applied to the fixing member 150 is disappears as the fixing member 150 passes through the second fixing hole 114 in the process of the fixing unit 130 retreating, the fixing member 150 is inserted into and fixed to the second fixing hole 114, and a region of the fixing member 150 is exposed to the outside of the second fixing hole 114. Then, the retreated state of the fixing unit 130 is maintained.

The first plunger 140 may include a coupling portion for engaging with the syringe 10 (specifically, the hub) in the second through hole 141. For example, the coupling portion may be a spiral portion for engaging in a rotating manner or a projection or groove for engaging in a fitting manner. Thus, coupling between the syringe 10 and the fixing unit 130 may be enhanced.

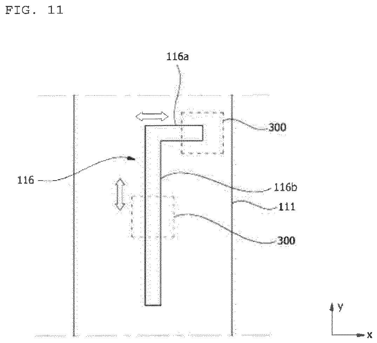

In order to enhance coupling between the syringe in 10 and the safety assembly 100, a guide rail 116 (see FIG. 11) extending along the central axis C may be provided to the cover 111. In addition, the cover 111 may be provided with a fixing body 300 arranged to be movable along the guide rail 116 and configured to engage with the housing 13 of the syringe 10.

The cover 110 may include a sealing member 200 arranged to allow the needle 11 to pass through the first through hole 111. The sealing member 200 may be formed of a material having high elasticity, high airtightness, and high corrosion resistance. For example, the sealing member 200 may be formed of a rubber material having high elasticity such as silicone or urethane. In addition, the sealing member 200 may be formed of a superabsorbent polymer whose volume expands by moisture. For example, the sealing member 200 may be formed of a copolymer of acrylic acid and vinyl alcohol and a copolymer of cellulose and acrylonitrile. In summary, the sealing member 200 may be formed of one or more selected from the group consisting of silicone, a rubber material, a copolymer of acrylic acid and vinyl alcohol, and a copolymer of cellulose and acrylonitrile.

The sealing member 200 is arranged to seal the first through hole 111 with the needle retreating into and accommodated in the cover 110.

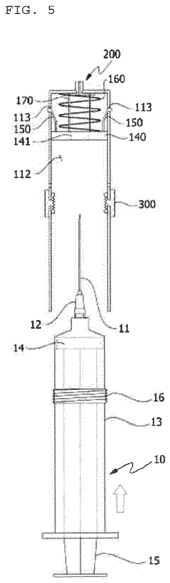

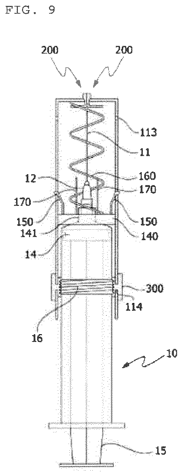

FIGS. 5 to 10 are cross-sectional views illustrating an operational state of the safety syringe 1 shown in FIG. 1, and FIG. 11 is a plan view illustrating the guide rail 116 and the fixture 300. As described above, the safety syringe 1 includes a syringe-coupled safety assembly 100 and a syringe 10. In the present invention, the syringe-coupled safety assembly 100 is applicable to the conventional syringe 10 having various structures with the needle 11.

Referring to FIGS. 5 and 11, the guide rail 116 extending along the central axis may be provided to the cover 111, and the cover 111 may be provided with the fixture 300 arranged to be movable along the guide rail 116 and provided with a first spiral portion for engaging with the housing 13 of the syringe 10. In addition, the housing 10 may be provided with a second spiral portion 16 to be screw-coupled to the first spiral portion. For example, the first spiral portion of the fixture 300 and the second spiral portion 16 may be a male thread and a female thread, or vice versa. The formation position of the second spiral portion 16 and the arrangement position of the fixture 300 may be variously determined. Specifically, the second spiral portion 16 may be provided at a position where it does not interfere with gradations or various markings provided to the housing 13. In addition, the position of the second spiral portion 16 may be differently determined according to the amount (small amount or large amount) of blood or a medical solution to be suctioned into or administered through the syringe 10. In addition, the fixture 300 may be arranged to correspond to the position of the second spiral portion 16.

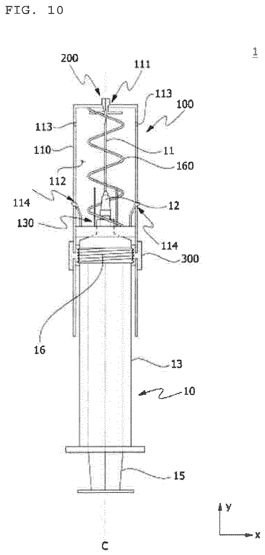

For example, referring to FIG. 10, the second spiral portion 16 of the housing 10 may be provided to the leading end portion of the housing 10. In this case, the fixture 300 may be provided at a position where the fixture 300 can be engaged with the second spiral portion 16.

Referring to FIG. 5, the syringe 10 may be inserted into and fixed to the syringe-attached safety assembly 100 with the fixing unit 130 in the advanced state by the user. The fixture 300 may be coupled to the second spiral portion 16 by inserting the hub 12 of the syringe 10 into the second through hole 141 and rotating the syringe or the safety assembly 100.

Of course, even when the fixing unit 130 is in the retreated state, the fixing unit 130 may be switched to the advanced state and the syringe 10 and the safety assembly 100 may be coupled to each other by causing the syringe 10 to advance while inserting the hub 12 of the syringe 10 into the second through hole 141. Specifically, in the state shown in FIG. 10, when coupling between the second spiral portion 16 of the housing 13 and the fixture 300 is completed, the fixing unit 130 may be switched to the advanced state by advancing the syringe 10 to compress the elastic member 160 and inserting the engagement member 150 into the first fixing hole 113 so as to be fixed. Thereafter, when the procedure using the syringe 10 is completed, the engagement member 150 may be separated from the first fixing hole 113 by pressing the engagement member 150 such that the syringe 10 retreats and the needle 11 also retreats and is positioned inside the cover 11. In addition, when only the advanced state of the syringe 10 (or the fixing unit) is desired to be fixed, the cover 110 may have only the first fixing hole 113 and retreat of the syringe 10 may be caused by the restoring force of the elastic member 170 alone without the second fixing hole 114.

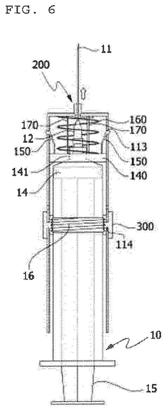

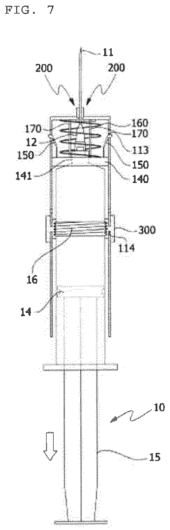

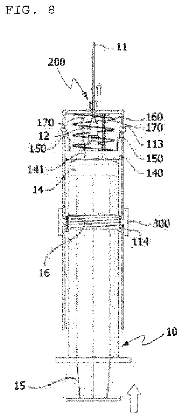

Referring to FIGS. 6 to 8, the needle 11 may be exposed to the outside of the safety assembly 100 through the sealing member 200, and the user may perform a procedure such as suction of a medical solution, blood sampling (see FIG. 7) or administration of a medical solution (see FIG. 8) by manipulating the plunger rod 15 of the syringe 10.

The guide rail 116 may include a first guide portion 116b extending along the central axis of the cover 111 and a second guide portion 116a extending in a circumferential direction of the cover 111. Specifically, the second guide portion 116a may extend from a terminal end of the first guide portion 116b so as to be perpendicular to the first guide portion 116b.

In this case, when the fixture 300 is positioned at the first guide portion 116b, the fixture is movable along the central axis of the cover 111. In contrast, when the fixture 300 is positioned at the second guide portion 116a, the fixture 300 is not allowed to move along the central axis of the cover 111. That is, in order to prevent the syringe 10 from moving back and forth during the procedure, the fixture 300 is positioned at the second guide portion 116a with the fixing unit 130 in the advanced state, as shown in FIGS. 7 and 8. That is, the user can couple the syringe 10 with the fixture 300 and then rotate the syringe 10 to position the fixture 300 at the second guide portion 116a with the fixing unit 130 in the advanced state.

When the fixing unit 130 retreats together with the syringe 10, the fixture 300 engaged with the syringe 10 must retreat as well. Accordingly, after the procedure is completed, the user can position the fixture 300 at the first guide portion 116b by rotating the syringe 10.

Hereinafter, the sealing member 200 will be described in detail with reference to the accompanying drawings.

The syringe-coupled safety assembly 100 includes a sealing member 200 provided in the first through hole 111 and configured to allow the needle 11 of the syringe 10 to pass therethrough. When the needle 11 passes through the sealing member 200 while the syringe 10 advances, the sealing member is moved in the advancing direction along the first through hole 111 (see FIG. 8). When the needle 11 passes through the sealing member while the syringe 10 retreats, the sealing member 200 is moved along the first through hole 111 in the retreating direction (see FIG. 4). In addition, when the needle 11 passes through the sealing member as the syringe 10 (or the fixing unit 130) retreats, the sealing member 200 seals the first through hole 111.

Specifically, the sealing member 200 has an opening 230 allowing the needle 11 to pass therethrough. The diameter of the opening 230 may vary depending on the advancing and retreating movements of the sealing member 200.

The sealing member 200 includes a sealing portion 220 and an engagement portion 210. The engagement portion 210 may extend from the sealing portion 220 at a predetermined angle. For example, the engagement portion 210 may be arranged to be perpendicular to the sealing portion 220. The engagement portion 210 may have an insertion hole 211.

Specifically, each sealing member 200 may include a sealing portion 220 including an opening 230 allowing the needle 11 of the syringe 10 to pass therethrough and a groove portion 221 extending along the central axis C of the cover 110 and arranged to contact the inner circumferential surface of the first through hole 111, and an engagement portion 210 extending from the sealing portion 220 and selectively contacting the inner circumferential surface of the cover 111.

The sealing portion 220 may have a cylindrical shape of a hollow (opening). The thickness of the sealing portion 220 may be varied along the central axis C of the cover 110. That is, the thickness of the sealing portion at the groove portion 221 is less than the thickness of the other regions. The engagement portion 210 may have a plate shape (circular shape, elliptical shape, or polygonal shape) having a diameter greater than that of the sealing part 220. In addition, the sealing portion 220 may be integrated with the engagement portion 210. The opening 230 is formed to integrally penetrate the sealing portion 220 and the engagement portion 210.

In addition, the sealing portion 220 may be provided with a first inclined surface 223 for guiding entry of the needle 11 when the syringe 10 advances. Referring to FIG. 3 and 6, when the needle 11 enters, the sealing portion 220 may advance along the central axis C of the cover 111, and be moved toward the inner circumferential surface of the first through hole 111. At this time, the diameter of the opening 230 of the sealing member 200 may be increased to some extent, and the needle 11 may easily enter the opening 230. At this time, if the engagement portion 210 contacts the inner circumferential surface of the cover 111, the advancing movement of the sealing portion 220 is restricted.

The sealing portion 220 may have a second inclined surface 222 in the groove portion 221. The inner circumferential surface of the first through hole 111 may be provided with a third inclined surface 115 matching (shape-matching) the second inclined surface. In this case, when the needle 11 retreats, and the second inclined surface 222 and the third inclined surface 115 are brought into contact with each other, the retreating movement of the sealing portion 220 may be restricted. In addition, as the sealing portion 220 retreats and the second and third inclined surfaces 223 and 115 are brought into contact with each other, the sealing portion 220 is pressed toward the central axis by the inner circumferential surface of the first through hole 111. As the diameter of the opening 230 is reduced, the sealing portion 220 seals the first through hole 111.

In order to assist the retreating movement of the sealing portion 220, the fixing unit 130 may include one or more latching members 170 connected to the first plunger 140 and selectively inserted into the sealing member 200. A plurality of latching members 170 may be provided to stably guide retreat of the sealing portion 220.

The latching members 170 may be inserted into the engagement portion 210 of the sealing member 200. Specifically, the latching members 170 may be inserted into the insertion hole 211 of the engagement portion 210 described above. In this case, the latching members 170 may be inserted into the insertion hole 211 in a fitting manner. When the latching members 170 retreat, the sealing member 200 may be caused to retreat through predetermined friction between the insertion hole 211 and the latching members 170. In addition, the latching members 170 may have a structure in which the latching members 170 are engaged with the insertion hole 211. Accordingly, when the fixing unit 130 retreats, the retreating movement of the engagement portion 210 may be caused by the latching members 170. In addition, when the needle 11 retreats into the cover 110, namely, when the needle 11 is separated from the sealing member 200, the first through hole 111 may be sealed by the sealing member 200. That is, the diameter of the opening 230 is reduced by contact between the second inclined surface 222 and the third inclined surface 115.

While the sealing member 200 is illustrated as being formed by a single member, the sealing member 200 may be divided into a plurality of sealing segments (e.g., one pair of sealing segments) arranged in the circumferential direction with respect to the center axis C of the cover 110. Each of the sealing segments may include a sealing portion 220 and an engagement portion 210 which are segmented in the circumferential direction with respect to the central axis C of the cover 110.

Although some embodiments of the present invention have been disclosed for illustrative purposes, it is to be understood that the invention is not limited to the disclosed exemplary embodiments. Those skilled in the art will appreciate that various modifications, variations and additions can be made to the present invention, without departing from the scope and spirit of the invention as disclosed in the accompanying claims.

### [Industrial Applicability]

In accordance with the present invention, a syringe-coupled safety assembly may be selectively connected to and disconnected from a syringe, depending on a use status of the syringe in order to improve usability.

Therefore, injury and infection caused by the syringe and needle may be prevented.

## Claims

1. A syringe-coupled safety assembly comprising:
a cover having a space for entry of a syringe and a first through hole allowing a needle of the syringe to pass therethrough; and
a fixing unit having a second through hole into which a hub of the syringe equipped with the needle is inserted, the fixing unit being disposed inside the cover so as to advance toward the first through hole and retreat from the first through hole and configured to be fixed to the cover in an advanced state and cause the syringe inserted into the second through hole to retreat when released from a fixed state with respect to the cover in the advanced state,
wherein the cover comprises a sealing member configured to allow the needle to pass through the first through hole,
wherein the sealing member comprises:
a sealing portion comprising an opening allowing the needle of the syringe to pass therethrough and a groove portion extending along a central axis of the cover and arranged to contact an inner circumferential surface of the first through hole; and
an engagement portion extending from the sealing portion and selectively contacting an inner circumferential surface of the cover.

2. The syringe-coupled safety assembly according to claim 1, wherein, when the fixing unit is in the advanced state, the needle of the syringe is exposed to an outside through the first through hole,
wherein, when the fixing unit is in a retreated state, the needle of the syringe is positioned inside the cover through the first through hole.

3. The syringe-coupled safety assembly according to claim 1, wherein the fixing unit comprises:
a first plunger having a second through hole and sealingly and slidably disposed in the cover;
one or more fixing members connected to the first plunger and selectively fixed to the cover; and
an elastic member disposed between the first plunger and the first through hole.

4. The syringe-coupled safety assembly according to claim 3, wherein the cover comprises, respectively:
a first fixing hole into which the fixing members are inserted in the advanced state of the fixing unit; and
a second fixing hole into which the fixing members are inserted in a retreated state of the fixing unit.

5. The syringe-coupled safety assembly according to claim 3, wherein the elastic member is fixed to the cover and the first plunger, respectively,
wherein the elastic member is configured to exert restoring force in a direction in which the fixing unit retreats.

6. The syringe-coupled safety assembly according to claim 4, wherein the fixing members have at least one region bent to exert elastic force in a radial direction with respect to the central axis of the cover.

7. The syringe-coupled safety assembly according to claim 6, wherein, when the fixing members are inserted into and fixed to the first fixing hole, a region of the fixing members is exposed to an outside of the first fixing hole,
wherein, when external force is applied in the radial direction, the fixing members are separated from the first fixing hole and entire regions of the fixing members are positioned inside the cover.

8. The syringe-coupled safety assembly according to claim 3, wherein the first plunger comprises a coupling portion in the second through hole to engage with the syringe.

9. The syringe-coupled safety assembly according to claim 8, wherein the coupling portion is a spiral portion for engaging in a rotating manner, or a projection or groove for engaging in a fitting manner.

10. The syringe-coupled safety assembly according to claim 1, wherein the sealing member is formed of one or more selected from the group consisting of silicone, a rubber material, a copolymer of acrylic acid and vinyl alcohol, and a copolymer of cellulose and acrylonitrile.

11. The syringe-coupled safety assembly according to claim 1, wherein the cover comprises a guide rail extending along the central axis,
wherein the cover is provided with a fixture arranged to be movable along the guide rail and configured to be engaged with a housing of the syringe.

12. A safety syringe comprising:
a syringe comprising a housing, a hub mounted on the housing, a needle mounted on the hub, a plunger disposed in the housing, and a plunger rod connected to the plunger; and
a syringe-coupled safety assembly detachably mounted on the syringe to protect the needle from an outside,
wherein the syringe-coupled safety assembly comprises:
a cover having a space for entry of the syringe and a first through hole allowing the needle of the syringe to pass therethrough; and
a fixing unit having a second through hole into which the hub of the syringe equipped with the needle is inserted, the fixing unit being disposed inside the cover so as to advance toward the first through hole and retreat from the first through hole and configured to be fixed to the cover in an advanced state and cause the syringe inserted into the second through hole to retreat when released from a fixed state with respect to the cover in the advanced state,
wherein the cover comprises a sealing member configured to allow the needle to pass through the first through hole,
wherein the sealing member comprises:
a sealing portion comprising an opening allowing the needle of the syringe to pass therethrough and a groove portion extending along a central axis of the cover and arranged to contact an inner circumferential surface of the first through hole; and
an engagement portion extending from the sealing portion and selectively contacting an inner circumferential surface of the cover.

13. The safety syringe according to claim 12, wherein the cover comprises a guide rail extending along the central axis,
wherein the cover is provided with a fixture arranged to be movable along the guide rail, the fixture comprising a first spiral portion for engaging with the housing of the syringe,
wherein the housing is provided with a second spiral portion for screw-coupling with the first spiral portion.

14. A syringe-coupled safety assembly comprising:
a cover having a space for entry of a syringe and a first through hole allowing a needle of the syringe to pass therethrough;
a sealing member provided in the first through hole, the sealing member being configured to allow the needle of the syringe to pass therethrough; and
a fixing unit having a second through hole into which a hub of the syringe equipped with the needle is inserted, the fixing unit being disposed inside the cover so as to advance toward the first through hole and retreat from the first through hole and configured to be fixed to the cover in an advanced state according to advance of the syringe in the space and cause the syringe to retreat when released from a fixed state with respect to the cover in the advanced state,
wherein the sealing member is configured to move along the first through hole in an advancing direction when the needle passes therethrough during advance of the syringe and to move along the first through hole in a retreating direction when the needle passes therethrough during retreat of the syringe,
wherein the sealing member comprises:
a sealing portion comprising an opening allowing the needle of the syringe to pass therethrough and a groove portion extending along a central axis of the cover and arranged to contact an inner circumferential surface of the first through hole; and
an engagement portion extending from the sealing portion and selectively contacting an inner circumferential surface of the cover.

15. The syringe-coupled safety assembly according to claim 14, wherein the sealing portion is provided with a first inclined surface for guiding entry of the needle when the syringe advances,
wherein, when the needle makes the entry, the sealing portion advances along the central axis of the cover, and is moved toward an inner circumferential surface of the first through hole.

16. The syringe-coupled safety assembly according to claim 14, wherein, when the engagement portion contacts an inner circumferential surface of the cover, an advancing movement of the sealing portion is restricted.

17. The syringe-coupled safety assembly according to claim 14, wherein the sealing portion is provided with a second inclined surface in the groove portion,
wherein the inner circumferential surface of the first through hole is provided with a third inclined surface shape-matching the second inclined surface,
wherein, when the needle retreats and the second inclined surface and the third inclined surface are brought into contact with each other, a retreating movement of the sealing portion is restricted.

18. The syringe-coupled safety assembly according to claim 14, wherein the fixing unit comprises:
a first plunger having a second through hole and sealingly and slidably disposed in the cover;
one or more fixing members connected to the first plunger and selectively fixed to the cover;
one or more latching members connected to the first plunger and selectively inserted into the sealing member; and
an elastic member disposed between the first plunger and the first through hole.

19. The syringe-coupled safety assembly according to claim 18, wherein a retreating movement of the engagement portion is caused by the latching members in accordance with a retreating movement of the fixing unit.

20. The syringe-coupled safety assembly according to claim 14, wherein, when the needle retreats into the cover, the first through hole is sealed by the sealing member.
